Europäisches Patentamt

European Patent Office

Office européen des brevets

(11) Veröffentlichungsnummer: **0 491 129 A2**

## EUROPÄISCHE PATENTANMELDUNG

(21) Anmeldenummer: **91117613.9**

(22) Anmeldetag: **16.10.91**

(51) Int. Cl.5: **C07C 255/04**, C07C 253/22

(30) Priorität: **17.12.90 DE 4040253**

(43) Veröffentlichungstag der Anmeldung:
**24.06.92 Patentblatt 92/26**

(84) Benannte Vertragsstaaten:
**AT BE CH DE FR GB IT LI NL SE**

(71) Anmelder: **HÜLS AKTIENGESELLSCHAFT
Patentabteilung / PB 15 - Postfach 13 20
W-4370 Marl 1(DE)**

(72) Erfinder: **Schmitz, Karl, Dr.
Ludwig-Bette-Weg 1
W-4390 Gladbeck(DE)**

(54) Verfahren zur Nitrilierung aliphatischer Dicarbonsäuren in der Flüssigphase.

(57) Die Erfindung betrifft ein Verfahren zur Nitrilierung von aliphatischen Dicarbonsäuren in der Flüssigphase bei Temperaturen von etwa 200 bis 350 °C mit Ammoniak in Gegenwart von Phosphorsäure und/oder Polyphosphorsäure, welches dadurch gekennzeichnet ist, daß dem Reaktionsgemisch ein Adsorptionsmittel für Phosphorsäure bzw. Polyphosphorsäure oder deren Ammoniumsalze zugesetzt wird.

EP 0 491 129 A2

Die Erfindung betrifft ein vorteilhaftes Verfahren zur Flüssigphase-Nitrilierung aliphatischer Dicarbonsäuren, wobei neben Phosphorsäure bzw. Polyphosphorsäure als Katalysator noch zusätzlich ein Adsorptionsmittel eingesetzt wird.

Aliphatische Dinitrile haben als Ausgangsprodukte zur Herstellung der entsprechenden Diamine große wirtschaftliche Bedeutung erlangt. Sie sind leicht zugänglich durch Nitrilierung aliphatischer Dicarbonsäuren mit Ammoniak bei erhöhten Temperaturen in Gegenwart eines Dehydratisierungskatalysators. Die Nitrilierung thermostabiler Säuren erfolgt vorzugsweise in der Gasphase über einen Festbettkatalysator bei Temperaturen zwischen 300 und 550 °C. Bei der Umsetzung längerkettiger Säuren, bei denen mit steigender Temperatur in zunehmendem Maße Zersetzungsreaktionen auftreten, wird die Flüssigphase-Nitrilierung angewandt. Bei diesem Verfahren arbeitet man im Temperaturbereich zwischen 200 und 350 °C und leitet in Gegenwart eines Katalysators Ammoniak in die als Schmelze vorliegende Säure (D. T. Mowry, "The Preparation of Nitriles", Chem. Rev. 42 (1948), 191 - 285). Die Flüssigphase-Nitrilierung bevorzugt Katalysatoren auf Basis der Phosphorsäure. Nach der deutschen Patentschrift 734 558 verwendet man zur Herstellung von Adipinsäuredinitril Phosphorsäure oder neutrale oder saure Phosphorsäureester. Man erhält aus Adipinsäure in einer Ausbeute von maximal 86,6 % der Theorie Adipinsäuredinitril. Die in dem Verfahren eingesetzten Katalysatoren haben beachtliche Nachteile. Phosphorsäure geht durch Wasserabspaltung in Polyphosphorsäure über und lagert sich im Reaktor ab. Dies führt zu einer Behinderung der Wärmezuführung und verursacht darüber hinaus Korrosionen. Die ebenfalls in dieser Patentschrift genannten Phosphorsäureester ergeben die gleichen Probleme, da diese unter den Nitrilierungsbedingungen hydrolysieren.

Gemäß der deutschen Patentschrift 857 194 wird ein Verfahren zur Herstellung von Dinitrilen aliphatischer alpha,omega-Dicarbonsäuren in Gegenwart von Phosphorsäure oder deren Salzen beschrieben. In Gegenwart von Metaphosphorsäure wird Sebazinsäure in einer Ausbeute von 82,1 % der Theorie zu Sebazinsäuredinitril umgesetzt. Die bei diesem Verfahren angewandten Katalysatoren Phosphorsäure, Metaphosphorsäure oder deren Salze führen auch hier zu den bereits genannten Schwierigkeiten.

Als weiteres Beispiel für die zahlreichen Nitrilierungsverfahren von Dicarbonsäuren in Gegenwart von Phosphorsäure wird die US-Patentschrift 3 297 740 genannt. Roh-Trimethyladipinsäure wird mit Ammoniak in einem Rührreaktor in Gegenwart von Phosphorsäure oder Phosphorsäuresalzen bei Temperaturen zwischen 240 und 290 °C zu Trimethyladipinsäuredinitril umgesetzt unter gleichzeitigem Abdestillieren der Reaktionsprodukte. Als Kopfprodukte fallen Ammoniak-haltiges Wasser und leichter flüchtige Nebenprodukte, insbesondere Trimethylcyclopentanon, an. Trimethyladipinsäuredinitril wird über einen Seitenabzug abgeleitet.

Roh-Trimethyladipinsäuredinitril enthält u. a. Trimethyladipinsäureimid. Bei der anschließenden Ammoniak-Wäsche entsteht daraus das entsprechende Diamid, welches dem Reaktor zugeführt und quantitativ in Trimethyladipinsäuredinitril umgesetzt wird. Bevor Trimethyladipinsäuredinitril im Vakuum destilliert wird, müssen in einer Natronlauge-Wäsche die Imide der aus der Roh-Trimethyladipinsäure stammenden kürzerkettigen Säuren entfernt werden. Auch bei diesem Nitrilierungs-Verfahren bilden sich Polyphosphorsäure-Ablagerungen, die Betriebsstörungen und Produktionsausfall verursachen.

Die Aufgabe der vorliegenden Erfindung bestand demnach darin, ein Verfahren zu entwickeln, nach dem höherkettige Dicarbonsäuren in Gegenwart eines Katalysatorsystems, welches zu keinen Ablagerungen und Korrosionen führt, mit Ammoniak in der Flüssigphase nitriliert werden können.

Überraschenderweise wurde nun gefunden, daß die bei der Flüssigphase-Nitrilierung von Dicarbonsäuren in Gegenwart von Phosphorsäure, Polyphosphorsäure und Metaphosphorsäure auftretenden Ablagerungen von Polyphosphorsäure an den Reaktorwandungen dadurch verhindert werden, daß dem Reaktionsgemisch ein Adsorptionsmittel zugegeben wird.

Gegenstand der Erfindung ist daher ein Verfahren zur Nitrilierung von aliphatischen Dicarbonsäuren in der Flüssigphase bei Temperaturen von 200 bis 350 °C mit Ammoniak in Gegenwart von Phosphorsäure und/oder Polyphosphorsäure, dadurch gekennzeichnet, daß dem Reaktionsgemisch ein Adsorptionsmittel für Phosphorsäure bzw. Polyphosphorsäure oder deren Ammoniumsalze zugesetzt wird.

Die Phosphorsäure bzw. Polyphosphorsäure schlägt sich nun nicht mehr an der Reaktor-Oberfläche, sondern auf dem Adsorptionsmittel nieder, ohne ihre Katalysatoraktivität zu verlieren. Nach Durchführung der Nitrilierung kann das Katalysatorsystem, bestehend aus Adsorptionsmittel und Phosphorsäure bzw. Polyphosphorsäure, leicht abgetrennt und erneut eingesetzt werden. Es besteht aber auch die Möglichkeit, das Reaktionsgemisch ohne Abtrennung des Katalysators durch Vakuumdestillation aufzuarbeiten und den Destillationsrückstand als Katalysatormasse für den nächsten Ansatz zu verwenden. Um den Destillationsrückstand nicht zu stark anwachsen zu lassen, sollte ein Teil davon ausgeschleust und das Katalysatorsystem entsprechend ergänzt werden. Während der Nitrilierung tritt eine gewisse Verkokung des Katalysatorsystems ein, die umso mehr ansteigt, je öfter der Katalysator in den Nitrilierungsprozeß zurückgeführt wird.

2

Die Verkokung hat zur Folge, daß die Katalysatoraktivität zunächst zunimmt, bei Überschreitung eines gewissen Verkokungsgrades aber stark abnimmt. Das Nachlassen der Katalysatoraktivität macht sich bemerkbar durch verminderte Wasserbildung. Nach Phosphorsäure-Zugabe wird die ursprüngliche Aktivität wieder erreicht.

Anstelle von Phosphorsäure, Metaphosphorsäure und Polyphosphorsäure lassen sich auch ihre Ammoniumsalze in Kombination mit den erfindungsgemäß eingesetzten Adsorptionsmitteln verwenden.

Geeignete Adsorptionsmittel sind Filterhilfsmittel auf Basis von Kieselgur und Tonsil-Bleicherden. Im allgemeinen genügen 50 bis 200 Gewichtsprozent Adsorptionsmittel, bezogen auf die eingesetzte Phosphorsäure, um Katalysator-Ablagerungen zu verhindern.

Die aliphatischen Dicarbonsäuren können 4 bis 18 C-Atome enthalten und geradkettig oder verzweigt sein. Auch cycloaliphatische bzw. aliphatisch-cycloaliphatische Dicarbonsäuren sind geeignet. Bevorzugte Einsatzprodukte sind Trimethyladipinsäure und Sebazinsäure.

Das erfindungsgemäße Katalysatorsystem läßt sich zweckmäßig wie folgt zuführen:

1. Zugabe des vorher mit Phosphorsäure getränkten Adsorptionsmittels vor oder während der Aufheizphase.

2. Gleichzeitige oder nachfolgende Zugabe des Adsorptionsmittels und der Phosphorsäure in die bereits geschmolzene Dicarbonsäure.

3. Zugabe einer Suspension, bestehend aus dem Adsorptionsmittel und der wäßrigen Phosphorsäure, in die bereits geschmolzene Dicarbonsäure.

Das erfindungsgemäße Verfahren soll durch die nachfolgenden Beispiele näher erläutert werden.

Beispiel 1

3 000 kg Sebazinsäure füllt man in den 4,7-$m^3$-Rührreaktor der Nitrilierungsanlage. Unter gleichzeitigem Einleiten von Ammoniak-Gas wird die Reaktor-Temperatur auf 300 °C eingestellt. Während der Aufheizphase dosiert man als Katalysator eine Suspension, hergestellt aus 15 kg Kieselgur-Filterhilfe, 15 kg Phosphorsäure und 75 kg Wasser, in 1 bis 2 Stunden hinzu. Während der Versuchsdauer von 30 bis 35 Stunden leitet man Ammoniak-Kreisgas und Frisch-Ammoniakgas in einer Menge von 15 bis 30 $m^3$/h in den Reaktor. Das bei der Umsetzung entstehende Reaktionswasser wird mit dem überschüssigen Ammoniakgas ausgetragen. Nach Beendigung der Nitrilierung destilliert man das Sebazinsäuredinitril im Vakuum ab.

Ausbeute an Sebazinsäuredinitril = 96,0 % d. Th.

Gasfraktometrische Reinheit = 99,6 %

Im Reaktor konnte keine Phosphorsäure-Ablagerung festgestellt werden. Der bei der Vakuumdestillation anfallende Destillationsrückstand wird als Katalysator für den jeweils nachfolgenden Ansatz eingesetzt. Erst nach sechs Rückführungen mußten 1,7 kg Phosphorsäure nachdosiert werden, um die ursprüngliche Katalysatoraktivität wieder zu erreichen.

Beispiel 2

Die Umsetzung von Sebazinsäure erfolgte entsprechend den in Beispiel 1 genannten Versuchsbedingungen. Anstelle der Katalysator-Suspension wurden 15 kg Phosphorsäure als 75%ige wäßrige Lösung eingesetzt. Ausbeute und Produktionsqualität entsprachen den Werten in Beispiel 1, jedoch wurden Polyphosphorsäure-Ablagerungen festgestellt.

Beispiel 3

800 g Roh-Trimethyladipinsäure, die bei der Salpetersäureoxidation von Trimethylcyclohexanol in 88,5%iger Reinheit anfällt, erhitzt man in einer Glasapparatur auf 200 °C und versetzt dann die Schmelze unter kräftigem Rühren zunächst mit 4 g Kieselgur-Filterhilfsmittel und dann mit 4 g 85%iger Phosphorsäure-Lösung. Unter gleichzeitigem Einleiten von 50 l/h Ammoniakgas erhöht man die Temperatur in ca. 30 Minuten auf 260 °C. Dann steigert man die Ammoniak-Dosierung auf 200 l/h und hält die Reaktionstemperatur 7 Stunden auf 260 °C. Bei der Reaktion entweichen über Kopf Ammoniak-Wasser und organische Nebenprodukte, insbesondere Trimethylcyclopentanon, die im Abscheider voneinander getrennt werden. Anschließend wird Roh-Trimethyladipinsäuredinitril im Vakuum destilliert. Der dabei anfallende Destillationsrückstand wird ohne weitere Phosphorsäure-Zugabe als Katalysatormasse für den nachfolgenden Versuch eingesetzt. Die Versuchsergebnisse sind in Tabelle 1 enthalten.

Beispiel 4

Die Nitrilierung von Roh-Trimethyladipinsäure in Gegenwart von 4 g 85%iger Phosphorsäure-Lösung erfolgte entsprechend Beispiel 3. Bei den Rückführungsversuchen diente der Destillationsrückstand als Katalysatormasse für den nachfolgenden Versuch. Die Versuchsergebnisse sind der Tabelle 2 zu entnehmen.

Tabelle 1

| Ver-such | Ein-satz-prod. (g) | Katalysator | Abscheider Org. Phase (g) | Abscheider NH$_3$-Wasser (g) | Roh.-prod. (g) | Poly-phosphor-säure-Ablagerung | Vakuum-Destillation Dest. (g) | Gasfrakt. Zus.-Setzung Trimethyl-adipinsäure-dinitril (%) | Trimethyl-adipinsäure-imid (%) | Dest.-Rück-stand (g) |
|---|---|---|---|---|---|---|---|---|---|---|
| 1 | 800 | 4 g Kieselgur-Filterhilfe + 4 g 85%ige H$_3$PO$_4$-Lösung | 42,8 | 353 | 578 | keine | 507 | 84,6 | 11,2 | 56 |
| 2 | 800 | Dest.-Rückstand Versuch 1 | 51,5 | 378 | 616 | keine | 532 | 84,0 | 11,6 | 79 |
| 3 | 800 | Dest.-Rückstand Versuch 2 | 52,8 | 375 | 633 | keine | 526 | 84,4 | 11,3 | 101 |
| 4 | 800 | Dest.-Rückstand Versuch 3 | 56,9 | 388 | 651 | keine | 525 | 84,7 | 11,0 | 121 |

Tabelle 2

| Ver-such | Ein-satz-prod. (g) | Katalysator | Abscheider Org. Phase (g) | NH$_3$-Wasser (g) | Roh. prod. (g) | Poly-phos-phor-säure-Abla-gerung | Vakuum-Destillation Dest. (g) | Gasfrakt. Zus.-Setzung Trimethyl-adipinsäure-dinitril (%) | Trimethyl-adipinsäure-imid (%) | Dest.-Rück-stand (g) |
|---|---|---|---|---|---|---|---|---|---|---|
| 5 | 800 | 4 g 85%ige H$_3$PO$_4$-Lösung | 39,6 | 372 | 567 | Ablag. | 490 | 85,7 | 10,0 | 71 |
| 6 | 800 | Dest.-Rückstand Versuch 5 | 46,0 | 377 | 638 | Ablag. | 536 | 85,6 | 10,5 | 97 |
| 7 | 800 | Dest.-Rückstand Versuch 6 | 47,5 | 372 | 658 | Ablag. | 521 | 86,1 | 9,8 | 131 |
| 8 | 800 | Dest.-Rückstand Versuch 7 | 49,3 | 348 | 684 | Ablag. | 519 | 86,4 | 9,5 | 161 |

**Patentansprüche**

1. Verfahren zur Nitrilierung von aliphatischen Dicarbonsäuren in der Flüssigphase bei Temperaturen von etwa 200 bis 350 °C mit Ammoniak in Gegenwart von Phosphorsäure und/oder Polyphosphorsäure,

dadurch gekennzeichnet,
daß dem Reaktionsgemisch ein Adsorptionsmittel für Phosphorsäure bzw. Polyphosphorsäure oder deren Ammoniumsalze zugesetzt wird.

2.  Verfahren nach Anspruch 1,
dadurch gekennzeichnet,
daß als Adsorptionsmittel für Phosphorsäure bzw. Polyphosphorsäure Kieselgur-Filterhilfsmittel und/oder Tonsil-Bleicherden eingesetzt werden.

3.  Verfahren nach den Ansprüchen 1 und 2,
dadurch gekennzeichnet,
daß als Dicarbonsäure Sebazinsäure eingesetzt wird.

4.  Verfahren nach den Ansprüchen 1 und 2,
dadurch gekennzeichnet,
daß als Dicarbonsäure Roh-Trimethyladipinsäure eingesetzt wird.